# EUROPEAN PATENT APPLICATION

(11) **EP 3 730 100 A1**
(43) Date of publication of application: **28.10.2020**
(21) Application number: 18891312.3
(22) Date of filing: 19.12.2018
(51) Int. Cl.: A61F 7/12, A61F 7/10, A61F 7/00

(54) **LOW-TEMPERATURE TREATMENT DEVICE CAPABLE OF PREVENTING NEURAL DAMAGE**

(30) Priority: 19.12.2017 KR 20170175006
(71) Applicant: Cebika Inc., Uiwang-si, Gyeonggi-do 16006 (KR)
(72) Inventor: CHO, Byoung Chic, Seongnam-si, Gyeonggi-do 13582 (KR)
(74) Representative: Ström & Gulliksson AB
(86) International application number: PCT/KR2018/016236
(87) International publication number: WO 2019/124971

(57) **Abstract**

The present invention relates to a low-temperature treatment device capable of preventing nerve damage. The low-temperature treatment device according to one embodiment of the present invention can comprise: a cooling pocket (100) which is inserted and positioned in a rectum and which has a predetermined shape during expansion; and a circulation tube (200) which has a passage through which fluid flows in and out and which is inserted into the cooling pocket so as to circulate the fluid in an inner space of the cooling pocket.

## Description

### [Technical Field]

The present invention relates to a low-temperature treatment device capable of preventing nerve damage.

### [Background Art]

Prostate cancer is a representative prostate gland disease. In relation to prostate cancer, prostate gland extirpation is a standard surgical treatment method, and in general, portions of the prostate gland, seminal vesicle, and testicular duct are cut and extirpated at the same time. In particular, a surgical operation is performed using electrocautery, which cuts living tissues while simultaneously coagulating the cut living tissues using high-frequency electrical energy. In such instances, heat may be conducted to the vicinity of the prostate gland and damage the nerve tissues around the prostate gland.

Generally, many important nerves pass around the lower side of the urinary bladder, seminal vesicle, and prostate gland, and while hypogastric nerves (*i.e.,* sympathetic nerves) and splanchnic nerves (*i.e.,* parasympathetic nerves) constitute the pelvic plexus and extend downwards, they are branched into the urinary bladder, the seminal vesicle, the prostate gland, the pelvis muscles, the penis, *etc.*

In particular, the cavernous nerves of the penis (*i.e., nervi cavernosi penis*) that control erection are mainly connected to the opposite lower sides of the vicinity of the prostate gland. Generally, when heat of 40 degrees to 60 degrees or more is applied, there is a risk of damaging the nerves.

Further, womb extirpation may be performed for female people who have uterine fibroids and cervical cancers, and in this case, similar situations also occur. That is, in female people, important nerves pass around the lower side of the urinary bladder, womb, and vagina, and accordingly, dysuresia and the like may be caused as complications due to damage to nerves after the womb extirpation.

In order to prevent the above-described complications due to damage to the nerves, Korean Patent No. 10-1142715 discloses a cooling balloon catheter which is to be inserted into a rectum, but there are disadvantages in that it is difficult to control the amount of the fluid injected into the balloon and that cooling efficiency is not high due to a structure of exchanging heat through a separate refrigerant circulation tube located in an interior of the balloon.

Further, Japanese Patent No. 2795540 discloses a probe device which is to be inserted into a rectum, but there is a disadvantage in that it is also difficult to control the amount of the fluid injected into the balloon.
(Patent document 1) Korean Patent No. 10-1142715 (May 10, 2012)
(Patent document 2) Japanese Patent No. 2795540 (June 26, 1998)

### [Disclosure]

### [Technical Problem]

The present invention has been made in an effort to solve the above-described problems. Specifically, the present invention provides a cooling pocket that has a shape by which heat applied to nerves around a prostate gland or a womb can be absorbed at maximum efficiency during an operation such as prostate gland extirpation or womb extirpation, and a low-temperature treatment device that can relatively easily control an amount of a fluid injected at maximum cooling efficiency while not pressing a portion that is to be inserted into.

### [Technical Solution]

In accordance with an aspect of the present invention, there is provided a low-temperature treatment device including: a cooling pocket 100 inserted into and located in a rectum and having a predetermined shape during expansion thereof; and a circulation tube 200 having a passage, through which a fluid is introduced and discharged, and inserted into the cooing pocket to circulate the fluid in a space in an interior of the cooling pocket.

In an embodiment, the cooling pocket 100 may have a shape corresponding to a body part of a subject, into which the cooling pocket 100 is to be inserted, in which a prostate gland is in contact with the rectum.

In an embodiment, the cooling pocket 100 may include, during the expansion thereof; an elliptically shaped first surface 101; an elliptically shaped second surface 102 disposed to be parallel to the first surface 101 and having an area that is larger than the area of the first surface 101; and a connection surface 103 connecting the first surface 101 and the second surface 102.

In an embodiment, an internal diameter of the connection surface 103 may increase as it extends from the first surface 101 toward the second surface 102.

In an embodiment, a portion at which the first surface 101 and the connection surface 103 are in contact with each other and a portion at which the second surface 102 and the connection surface 103 are in contact with each other may be rounded.

In an embodiment, the first surface 101 may be inserted into the rectum earlier than the second surface 102, and the first surface 101 may be located on an inner side of the rectum relative to the second surface 102.

In an embodiment, the cooling pocket 100 may have a predetermined fluid capacity, and may have the predetermined shape when the fluid corresponding to the predetermined fluid capacity is stored in the cooling pocket 100.

In an embodiment, the predetermined shape may be maintained even if the fluid corresponding to an amount that is larger than the predetermined fluid capacity is injected into the cooling pocket 100.

In an embodiment, the circulation tube 200 may include: a first circulation tube 201 communicating with the cooling pocket 100; and a second circulation tube 202 which is bent while defining a predetermined angle (α) with respect to the first circulation tube 201 to communicate with the first circulation tube 201.

In an embodiment, the circulation tube 200 may include: a fluid injection tube 210 that is a passage, through which the fluid to be injected into the cooling pocket 100 flows; and a fluid discharge tube 220 that is a passage, through which the fluid injected into the cooling pocket 100 is discharged.

In an embodiment, an injection hole 210a to be communicated with the fluid injection tube 210 and a discharge hole 220a to be communicated with the fluid discharge tube 220 may be formed in the circulation tube 200 located in an interior of the cooling pocket 100; the fluid may be injected into the cooling pocket 100 through the injection hole 210a; and the fluid in the cooling pocket 100 may be discharged through the discharge hole 220a.

In an embodiment, the circulation tube 200 may further include: a temperature measurement tube 230, in which a temperature sensor 231 configured to detect a temperature of the fluid accommodated in the cooling pocket 100 is installed; and a pressure measurement tube 240, in which a pressure sensor 241 configured to detect a pressure of the fluid accommodated in the cooling pocket 100 is installed.

In an embodiment, the angle (α) defined by the first circulation tube 201 and the second circulation tube 202 may be 126 degrees to 154 degrees.

In an embodiment, the low-temperature treatment device may further include: a cooling bag 300, by which the fluid to be injected or discharged through the circulation tube 200 is cooled.

In an embodiment, the cooling bag 300 may include a cooling passage 310 which is bent a plurality of times; one end of the cooling passage 310 may communicate with the fluid injection tube 210; and an opposite end of the cooling passage 310 may communicate with the fluid discharge tube 220.

In an embodiment, the low-temperature treatment device may further include a peristaltic pump 400, which is located between the cooling bag 300 and the circulation tube 200 and which is configured to inject the fluid cooled by the cooling bag 300 into the cooling pocket 100 and which provides power for discharging the fluid accommodated in the cooling pocket 100.

In an embodiment, the low-temperature treatment device may further include a control device 500 configured to control the peristaltic pump 400.

In an embodiment, the control device 500 may include a display 510 configured to display a temperature and a pressure of the fluid accommodated in the cooling pocket 100; and a controller 520 configured to control the peristaltic pump 400.

Further, the present invention provides a low-temperature treatment device including: a cooling pocket 100, which is inserted into a rectum, located at a site that faces a rear surface of a prostate gland of a subject, into which the cooling pocket 100 is to be inserted, and which has a predetermined shape during expansion thereof; and a circulation tube 200, which has a passage, through which a fluid is introduced and discharged, and which is inserted into the cooling pocket to circulate the fluid in a space in an interior of the cooling pocket. The present invention provides the low-temperature treatment device, wherein the cooling pocket 100 has a predetermined fluid capacity and has the predetermined shape when the fluid corresponding to the predetermined fluid capacity is stored in the cooling pocket 100. The present invention provides the low-temperature treatment device, wherein the predetermined shape is maintained even if the fluid corresponding to an amount that is larger than the predetermined fluid capacity is injected into the cooling pocket 100. The present invention provides the low-temperature treatment device, wherein a surface of the predetermined shape, which faces a rear surface of the prostate gland, has a shape corresponding to the shape in which the prostate gland and the rectum are in contact with each other, wherein the predetermined shape includes: a first surface 101 located at a tip end thereof; a second surface 102 facing the first surface 101 and having an area that is larger than the area of the first surface 101; and a connection surface 103 connecting the first surface 101 and the second surface 102. The present invention provides the low-temperature treatment device, wherein insertion holes for inserting the circulation tube 200 are each formed on the first surface 101 and the second surface 102, wherein the circulation tube 200 is installed in the cooling pocket 100 after passing through the insertion holes formed on the first surface 101 and the second surface 102, and wherein the circulation tube 200 includes: a first circulation tube 201 communicating with the cooling pocket 100; and a second circulation tube 202 which is bent while defining a predetermined angle (α) with respect to the first circulation tube 201 to communicate with the first circulation tube 201.

### [Advantageous Effects]

The present invention as described above has the following effects.

First, nerve damage can be prevented by absorbing heat applied to nerves around a prostate gland during an operation such as prostate gland extirpation, *etc.*

Second, side-effects of the operation can be minimized by restraining an inflammatory response that may occur after the operation due to a low-temperature treatment.

Third, the cooling efficiency of tissues around the prostate gland can be maximized because one surface of the cooling pocket is provided to correspond to a shape in which the prostate gland and a rectum are in contact with each other.

Fourth, because the cooling pocket has a predetermined fluid capacity and is formed of a material having a repulsive force of preventing injection of the fluid corresponding to the fluid capacity or more, the fluid is excessively injected, and thereby the cooling pocket is prevented from pressing the rectum or the prostate gland.

Fifth, because the first circulation tube and the second circulation tube are bent, the cooling pocket can be easily inserted into the rectum, and the surrounding rectum tissues are not pressed even in the insertion state.

Sixth, because a temperature and pressure of the fluid accommodated in an interior of the cooling pocket can be measured and can be identified through a display, the convenience of the operation is improved.

### [Brief Description of Drawings]

FIG. 1 is an overall perspective view for illustrating the low-temperature treatment device according to an embodiment of the present invention.
FIG. 2 is a perspective view for illustrating a cooling pocket of the low-temperature treatment device of FIG. 1.
FIG. 3 illustrates drawings of FIG. 2, which are viewed from one side, wherein FIG. 3a is a side view of FIG. 1 and FIG. 3b is a front view of FIG. 1.
FIG. 4 is a perspective view for illustrating a circulation tube of the low-temperature treatment device of FIG. 1.
FIG. 5 is a cross-sectional view taken along line A-A' of FIG. 4.
FIG. 6 is a cross-sectional view taken along line B-B' of FIG. 4.
FIG. 7 is a drawing for illustrating the circulation tube of the low-temperature treatment device of FIG. 1.
FIG. 8 is a view illustrating a use state of the low-temperature treatment device according to the embodiment of the present invention.

### [Best Mode for Carrying Out the Invention]

Hereinafter, the present invention will be described in detail with reference to the accompanying drawings.

The present invention relates to a low-temperature treatment device which is a medical instrument. It is said in advance that the present invention does not relate to a treatment method in which a human body is not a direct target.

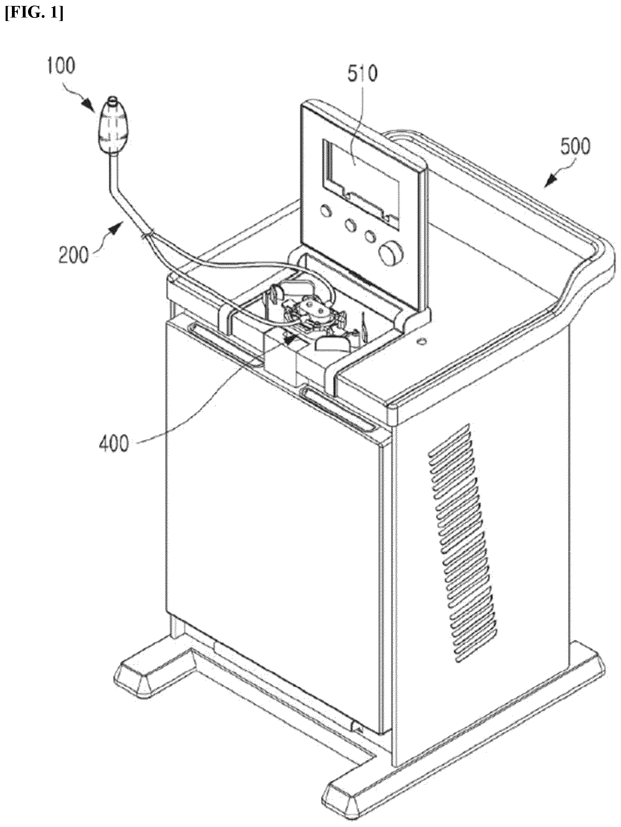

Hereinafter, for description, the upper side of FIG. 1 is referred to as an upper side, and the lower side of FIG. 1 is referred to as a lower side.

Referring to FIG. 1, the low-temperature treatment device according to an embodiment of the present invention includes a cooling pocket 100, a circulation tube 200, a cooling bag 300, a peristaltic pump 400, and a control device 500.

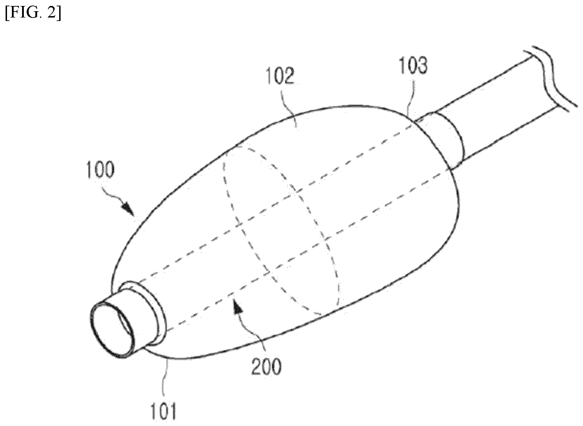

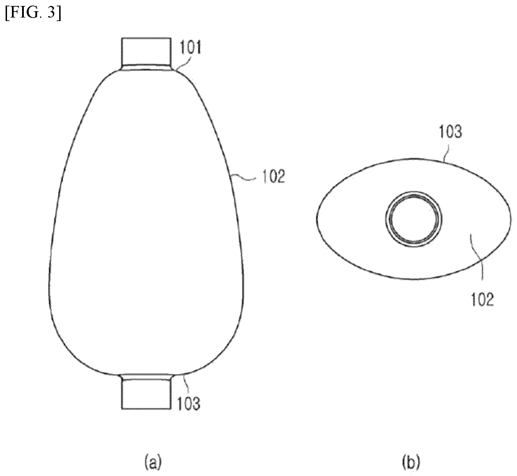

First, the cooling pocket 100 will be described in detail with reference to FIGS. 2 and 3.

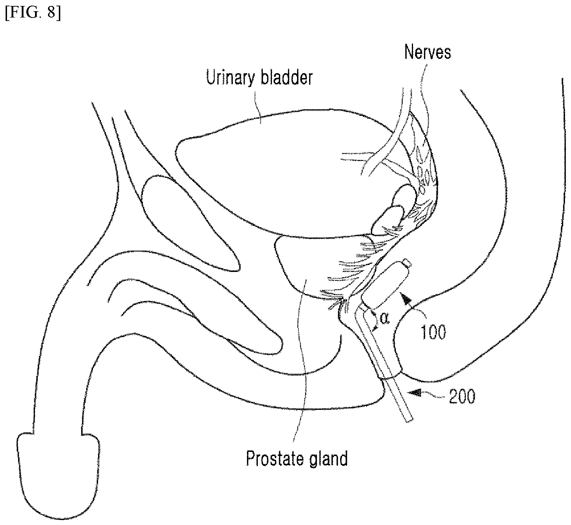

The cooling pocket 100 is a part that is inserted into and located in a rectum and prevents damage to nerves located around the cooling pocket 100 through circulation of a fluid, which is injected and discharged (see FIG. 8).

As illustrated in FIGS. 2 and 3, the cooling pocket 100 has a predetermined shape during expansion thereof. The cooling pocket 100 has a predetermined fluid capacity, and has the predetermined shape when the fluid corresponding to the fluid capacity is stored in the cooling pocket 100.

In particular, the important point is that the predetermined shape is maintained even if the fluid corresponding to an amount that is larger than the fluid capacity is injected into the cooling pocket 100. A conventional low-temperature treatment device uses a balloon, the shape of which changes according to the amount of the fluid injected.

Meanwhile, in the present invention, the shape of the balloon changes according to the amount of the fluid injected when the amount of the fluid injected is smaller than the fluid capacity, but the balloon has the predetermined shape when the amount of the fluid injected coincides with the fluid capacity, and the predetermined shape is maintained even if the fluid corresponding to an amount that is larger than the fluid capacity is injected. That is, it is preferable that the cooling pocket 100 be formed of a material having a predetermined elasticity to prevent injection of the fluid corresponding to the amount that is larger than the fluid capacity.

The shape of the cooling pocket 100 will be described in detail with reference to FIGS. 2 and 3. FIG. 2 is a perspective view of the cooling pocket 100. FIG. 3a is a cross-sectional view taken along line A-A' of FIG. 2, and FIG. 3b is a cross-sectional view taken along line B-B' of FIG. 2.

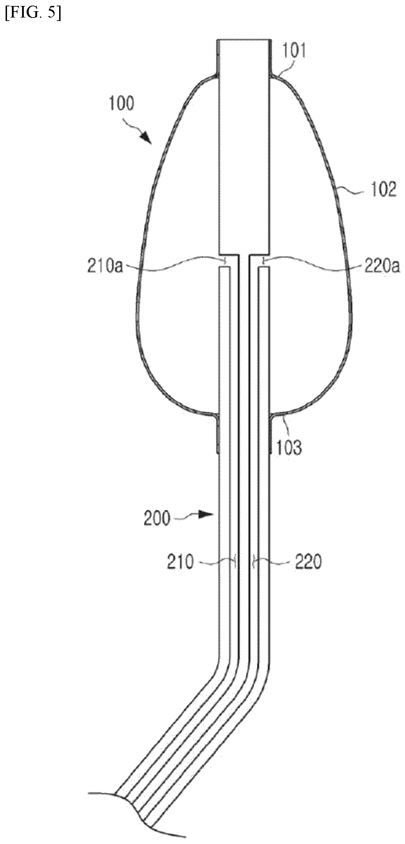

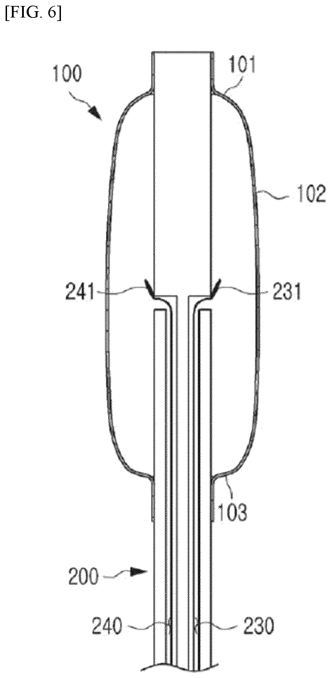

The cooling pocket 100 has, during the expansion thereof, an elliptically shaped first surface 101, an elliptically shaped second surface 102 disposed to be parallel to the first surface 101 and having an area that is larger than the area of the first surface 101, and a connection surface 103 connecting the first surface 101 and the second surface 102. As illustrated in FIGS. 5 and 6, the widths of the first surface 101 and the second surface 102 are the same, and the height of the second surface 102 is determined to be greater than the height of the first surface 101.

Further, insertion holes, into which the circulation tube 200 is inserted, are formed in each of the first surface 101 and the second surface 102.

Because the area of the second surface 102 is larger than the area of the first surface 101, the internal diameter of the connection surface 103 increases as it extends from the first surface 101 toward the second surface 102. Further, because the cooling pocket 100 is a part that is actually inserted into the rectum, it is preferable that the cooling pocket 100 be rounded as a whole to prevent scars from occurring in the tissues around the cooling pocket 100 during the insertion process, and in particular, it is preferable that a portion at which the first surface 101 and the connection surface 103 be in contact with each other, and that a portion at which the second surface 102 and the connection surface 103 are in contact with each other be rounded.

As such, one surface of the cooling pocket 100 (specifically, a side surface of the cooling pocket) has a shape corresponding to a body part of a subject, into which the cooling pocket 100 is to be inserted, in which the prostate gland is in contact with the rectum (see FIGS. 2, 3, and 8). Due to the shape, damage to the nerves that pass by the prostate gland can be efficiently prevented during the operation for the prostate gland.

As illustrated in FIG. 8, the first surface 101 of the cooling pocket 100 is inserted into the rectum first, and is located on an inner side of the rectum relative to the second surface 102.

Accordingly, as illustrated in FIG. 8, it is observed that the width of the cooling pocket 100 is uniform when the cooling pocket 100 is viewed from a side of a subject, into which the cooling pocket 100 is to be inserted, and it is observed that the width of the cooling pocket 100 increases toward the second surface 102 when the cooling pocket 100 is viewed from the front side (the shape of FIG. 5).

The circulation tube 200 is a part that has a passage, through which the fluid is introduced and discharged, and is inserted into the cooling pocket 100 to circulate the fluid in the space in the interior of the cooling pocket 100. The circulation tube 200 is inserted into the cooling pocket 100 through an insertion hole formed in the cooling pocket 100.

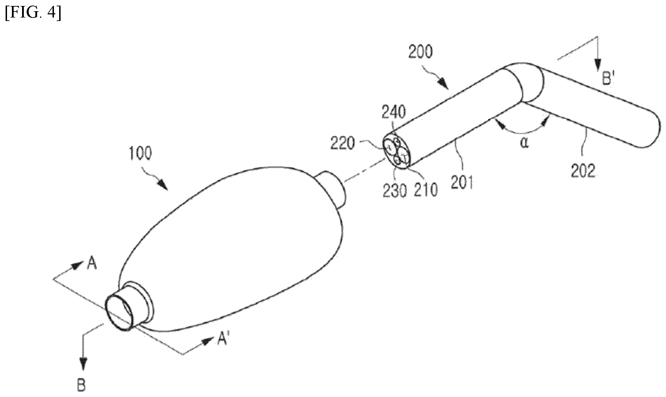

Referring to FIG. 4, the circulation tube 200 includes a first circulation tube 201 communicating with the cooling pocket 100, and a second circulation tube 202 which is bent while defining a predetermined angle (α) with respect to the first circulation tube 201 to communicate with the first circulation tube 201.

It is preferable that the predetermined angle (α) be 126 degrees to 154 degrees, and in particular, it is more preferable that the predetermined angle (α) be 140 degrees.

When the cooling pocket 100 is inserted into the rectum, the operator grips the circulation tube 200. Then, the cooling pocket 100 is inserted into the rectum while a force is applied in the direction of the cooling pocket 100, and the cooling pocket 100 can be inserted with less force while the circulation tube 200 is formed in a straight line.

Further, as illustrated in FIG. 8, the rectum is not formed in a straight line but is formed such that an entrance and an inner passage of the rectum define a predetermined angle. In order to maximize the cooling efficiency of the cooling pocket 100, it is important that the cooling pocket 100 be located immediately behind the prostate gland.

According to the present invention, because the first circulation tube 201 and the second circulation tube 202 are bent while defining a predetermined angle (α), the circulation tube 200 does not press the surrounding rectum tissues even if the cooling pocket 100 is located at the location above. That is, the structure is a structure that facilitates the insertion while minimizing side-effects thereof.

Referring to FIG. 4, a fluid injection tube 210, a fluid discharge tube 220, a temperature measurement tube 230, and a pressure measurement tube 240 pass through the interior of the circulation tube 200 in a lengthwise direction thereof.

The fluid is injected into the cooling pocket 100 through the fluid injection tube 210, and the fluid accommodated in the cooling pocket 100 is discharged through the fluid discharge tube 220.

The fluid has a temperature that is lower than a temperature of the subject, and the temperature of the fluid increases because the heat of the subject is transferred to the fluid over time. Accordingly, in order to maintain cooling efficiency, it is necessary to continuously circulate the fluid. This circulation is performed through the fluid injection tube 210 and the fluid discharge tube 220. The fluid discharged through the fluid discharge tube 220 is injected into a cooling bag 300, which will be described below, to be cooled.

An injection hole 210a, which communicates with the fluid injection tube 210, and a discharge hole 220a, which communicates with the fluid discharge tube 220, are formed in the circulation tube 200 located in the interior of the cooling pocket 100. The fluid cooled by the cooling bag 300 is discharged from the injection hole 210a through the fluid injection tube 210 to be injected into the cooling pocket 100, and the fluid, the heat of which has been transferred, is discharged from the fluid discharge tube 220 through the discharge hole 220a and injected into the cooling bag 300.

A temperature sensor 231 is installed in the temperature measurement tube 230 to measure a temperature of the fluid accommodated in the cooling pocket 100, and a pressure sensor 241 is installed in the pressure measurement tube 240 to measure a pressure of the fluid accommodated in the cooling pocket 100. The thus-measured temperature and pressure are displayed on a display 510 of a control device 500, and information that is helpful for an operation is provided to the operator.

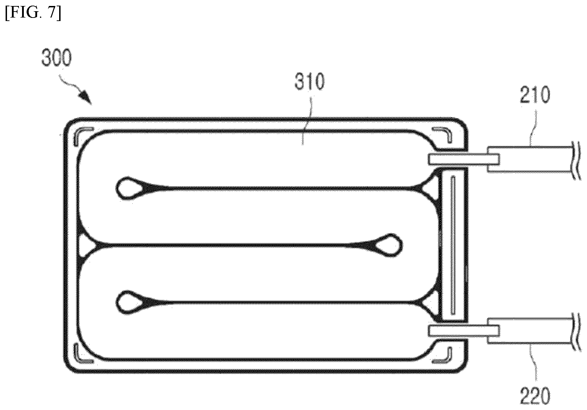

A cooling passage 310 is formed in the interior of the cooling bag 300. The cooling passage 310 communicates with the fluid injection tube 210 and the fluid discharge tube 220, and it is preferable that the cooling passage 310 be bent a plurality of times to achieve maximum cooling efficiency in the cooling bag 300. The bending angle is not limited, and the number of bends is also not limited. FIG. 7 is a view illustrating an embodiment of the cooling passage 310 which is bent three times while having an angle of 180 degrees.

In order to circulate the fluid, a part that provides circulation power is necessary. A peristaltic pump 400 performs this function, and the peristaltic pump 400 is located between the circulation tube 200 and the cooling bag 300, and the fluid injection tube 210 and the fluid discharge tube 220 are connected to the peristaltic pump 400. While the peristaltic pump 400 is operated, the fluid may circulate through the cooling bag 300, the fluid injection tube 210, the cooling pocket 100, the fluid discharge tube 220, and the cooling bag 300 in the order described.

The peristaltic pump 400 is controlled by the control device 500. The control device 500 is a part that provides electric power for operating the peristaltic pump 400, and includes a display 510 that displays a temperature and pressure of the fluid in the interior of the cooling pocket 100, and a controller 520 that controls the peristaltic pump 400.

As an example, the controller 520 may adjust the pumping speed of the peristaltic pump 400 to perform a pumping operation at a higher speed in the initial stage of the insertion to expand the cooling pocket 100, and to control the peristaltic pump 400 such that the pumping speed becomes slower than the initial speed after the expansion to make the fluid that removes heat of the tissues around the prostate gland flow.

The cooling bag 300 cools the fluid that is circulated by being accommodated in the interior of the control device 500. The fluid, its temperature having been increased by the cooling bag 300, is cooled again, followed by injection thereof into the cooling pocket 100 again.

Although the embodiments illustrated in the drawings have been described in the specification for reference such that a person skilled in the art can easily understand and realize the present invention, they are merely exemplary, and a person skilled in the art can understand that various modifications and equivalent embodiments are also possible from the embodiments of the present invention. Accordingly, the scope of the present invention is to be determined by the claims.

### (Description of Reference Numerals)

- 100:: cooling pocket
- 101:: first surface
- 102:: second surface
- 103:: connection surface
- 200:: circulation tube
- 210:: fluid injection tube
- 220:: fluid discharge tube
- 230:: temperature measurement tube
- 231:: temperature sensor
- 240:: pressure measurement tube
- 241:: pressure sensor
- 300:: cooling bag
- 310:: cooling passage
- 400:: peristaltic pump
- 500:: control device
- 510:: display
- 520:: controller
- 1000:: low-temperature treatment device

## Claims

1. A low-temperature treatment device comprising:
a cooling pocket (100) inserted into and located in a rectum and having a predetermined shape during expansion thereof; and
a circulation tube (200) having a passage, through which a fluid is introduced and discharged, and inserted into the cooling pocket to circulate the fluid in a space in an interior of the cooling pocket.

2. The low-temperature treatment device of claim 1, wherein the cooling pocket (100) has a shape corresponding to a body part of a subject, into which the cooling pocket (100) is to be inserted, in which a prostate gland is in contact with the rectum.

3. The low-temperature treatment device of claim 2, wherein the cooling pocket (100) comprises, during the expansion thereof;
an elliptically shaped first surface (101);
an elliptically shaped second surface (102) disposed to be parallel to the first surface (101) and having an area that is larger than the area of the first surface (101); and
a connection surface (103) connecting the first surface (101) and the second surface (102).

4. The low-temperature treatment device of claim 3, wherein an internal diameter of the connection surface (103) increases as it extends from the first surface (101) toward the second surface (102).

5. The low-temperature treatment device of claim 4, wherein a portion at which the first surface (101) and the connection surface (103) are in contact with each other and a portion at which the second surface (102) and the connection surface (103) are in contact with each other are rounded.

6. The low-temperature treatment device of claim 3, wherein the first surface (101) is inserted into the rectum earlier than the second surface (102) and the first surface (101) is located on an inner side of the rectum relative to the second surface (102).

7. The low-temperature treatment device of claim 3, wherein the cooling pocket (100) has a predetermined fluid capacity and has the predetermined shape when the fluid corresponding to the predetermined fluid capacity is stored in the cooling pocket (100).

8. The low-temperature treatment device of claim 7, wherein the predetermined shape is maintained even if the fluid corresponding to an amount that is larger than the predetermined fluid capacity is injected into the cooling pocket (100).

9. The low-temperature treatment device of claim 1, wherein the circulation tube (200) comprises:
a first circulation tube (201) communicating with the cooling pocket (100); and
a second circulation tube (202) which is bent while defining a predetermined angle (α) with respect to the first circulation tube (201) to communicate with the first circulation tube (201).

10. The low-temperature treatment device of claim 9, wherein the circulation tube (200) comprises:
a fluid injection tube (210) that is a passage, through which the fluid to be injected into the cooling pocket (100) flows; and
a fluid discharge tube (220) that is a passage, through which the fluid injected into the cooling pocket (100) is discharged.

11. The low-temperature treatment device of claim 10, wherein
an injection hole (210a) to be communicated with the fluid injection tube (210) and a discharge hole (220a) to be communicated with the fluid discharge tube (220) are formed in the circulation tube (200) located in an interior of the cooling pocket (100);
the fluid is injected into the cooling pocket (100) through the injection hole (210a); and
the fluid in the cooling pocket (100) is discharged through the discharge hole (220a).

12. The low-temperature treatment device of claim 10, wherein the circulation tube (200) further comprises:
a temperature measurement tube (230), in which a temperature sensor (231) configured to detect a temperature of the fluid accommodated in the cooling pocket (100) is installed; and
a pressure measurement tube (240), in which a pressure sensor (241) configured to detect a pressure of the fluid accommodated in the cooling pocket (100) is installed.

13. The low-temperature treatment device of claim 9, wherein the angle (α) defined by the first circulation tube (201) and the second circulation tube (202) is 126 degrees to 154 degrees.

14. The low-temperature treatment device of claim 10, further comprising:
a cooling bag (300), by which the fluid to be injected or discharged through the circulation tube (200) is cooled.

15. The low-temperature treatment device of claim 14, wherein the cooling bag (300) comprises a cooling passage (310) which is bent a plurality of times, one end of the cooling passage (310) communicates with the fluid injection tube (210), and an opposite end of the cooling passage (310) communicates with the fluid discharge tube (220).
